# EUROPEAN PATENT APPLICATION

(11) **EP 3 009 066 A1**
(43) Date of publication of application: **20.04.2016**
(21) Application number: 15186398.2
(22) Date of filing: 23.09.2015
(51) Int. Cl.: A61B 5/00, A61B 8/00

(54) **PHOTOACOUSTIC WAVE SIGNAL CONVERTER**

(30) Priority: 15.10.2014 JP 2014210719
(71) Applicant: PreXion Corporation, Tokyo 101-0041 (JP)
(72) Inventor: AGANO, Toshitaka, Chiyoda, Tokyo 101-0041 (JP)
(74) Representative: Grünecker Patent- und Rechtsanwälte PartG mbB

(57) **Abstract**

A photoacoustic wave signal converter (40) includes a receiving portion (41) that receives a photoacoustic wave signal generated from a detection object (Q) in a specimen (P) that has absorbed light, detected by an ultrasonic probe (20) and a signal conversion portion (42) that generates a conversion signal corresponding to an ultrasonic signal generated when the ultrasonic probe receives an ultrasonic wave on the basis of the photoacoustic wave signal received by the receiving portion and outputs the conversion signal that is generated to an ultrasonic imager (10).

## Description

### BACKGROUND OF THE INVENTION

### Field of the Invention

The present invention relates to a photoacoustic wave signal converter.

### Description of the Background Art

An ultrasonic imager that obtains an image of the inside of a specimen by ultrasonic waves reflected in the specimen is known in general, as disclosed in Japanese Patent Laying-Open No. 2001-104303.

Japanese Patent Laying-Open No. 2001-104303 discloses an ultrasonic diagnostic apparatus (ultrasonic imager) including a plurality of vibrating elements that emits ultrasonic pulses into a specimen and detects ultrasonic waves reflected in the specimen, a probe including the plurality of vibrating elements, and a signal processing portion that acquires received signals (ultrasonic signals) of the ultrasonic waves reflected in the specimen through the probe and obtains an image of the inside of the specimen by the reflected ultrasonic waves.

Furthermore, a photoacoustic imager is known as an apparatus that obtains an image of the inside of a specimen in general. This photoacoustic imager is configured to obtain the image of the inside of the specimen by applying light into the specimen through a surface of the specimen and detecting a photoacoustic wave (ultrasonic wave) generated from a detection object in the specimen. The photoacoustic wave denotes an ultrasonic wave generated by light absorption into the detection object in the specimen.

The ultrasonic diagnostic apparatus described in Japanese Patent Laying-Open No. 2001-104303 and the conventional photoacoustic imager are functionally similar in that both obtain images on the basis of ultrasonic waves, and hence the ultrasonic diagnostic apparatus conceivably also detects a photoacoustic wave to obtain an image.

However, the photoacoustic imager must generate a photoacoustic wave from a wide range at a time by light application and capture received signals (photoacoustic wave signals) of the photoacoustic wave in parallel (simultaneously) by a plurality of vibrating elements, unlike the ultrasonic diagnostic apparatus. Thus, a correct image cannot be displayed on the conventional marketed ultrasonic diagnostic apparatus when an image based on photoacoustic wave signals is intended to be displayed. In order to resolve this disadvantage, hardware incorporated in an apparatus body of the conventional ultrasonic diagnostic apparatus must be changed. Therefore, in the conventional marketed ultrasonic diagnostic apparatus (ultrasonic imager), it is desired to obtain the function of the photoacoustic imager and display the image based on the photoacoustic wave signals without changing the apparatus body hardware.

### SUMMARY OF THE INVENTION

The present invention has been proposed in order to solve the aforementioned problem, and an object of the present invention is to provide a photoacoustic wave signal converter allowing a marketed ultrasonic imager to obtain the function of a photoacoustic imager and to display an image based on photoacoustic wave signals without changing imager body hardware.

In order to attain the aforementioned object, a photoacoustic wave signal converter according to an aspect of the present invention includes a receiving portion that receives a photoacoustic wave signal generated from a detection object in a specimen that has absorbed light, detected by an ultrasonic probe and a signal conversion portion that generates a conversion signal corresponding to an ultrasonic signal generated when the ultrasonic probe receives an ultrasonic wave on the basis of the photoacoustic wave signal received by the receiving portion and outputs the conversion signal that is generated to an ultrasonic imager. According to the present invention, an ultrasonic wave generated by light absorption into the detection object in the specimen is referred to as a "photoacoustic wave", and an ultrasonic wave generated by the ultrasonic probe, reflected in the specimen is referred to as an "ultrasonic wave".

As hereinabove described, the photoacoustic wave signal converter according to the aspect of the present invention is provided with the signal conversion portion that generates the conversion signal corresponding to the ultrasonic signal generated when the ultrasonic probe receives the ultrasonic wave on the basis of the photoacoustic wave signal received by the receiving portion and outputs the conversion signal that is generated to the ultrasonic imager. Thus, even if the ultrasonic probe captures a received signal (photoacoustic wave signal) of a photoacoustic wave in parallel (simultaneously), the captured photoacoustic wave signal can be converted into the conversion signal corresponding to the ultrasonic signal generated when the ultrasonic probe receives the ultrasonic wave. Consequently, the conversion signal corresponding to the ultrasonic signal can be output to the ultrasonic imager, and hence the ultrasonic imager can process the conversion signal into which the photoacoustic wave signal is converted, substantially similarly to the case of processing the ultrasonic signal. Therefore, the marketed ultrasonic imager can serve as a photoacoustic imager and display an image based on the photoacoustic wave signal, using the photoacoustic wave signal converter without changing imager body hardware.

In the aforementioned photoacoustic wave signal converter according to the aspect, the signal conversion portion is preferably configured to generate the conversion signal on the basis of the photoacoustic wave signal received by the receiving portion and to output the conversion signal that is generated to the ultrasonic imager according to a reception cycle of the ultrasonic signal received by the ultrasonic imager. According to this structure, the ultrasonic imager can receive the conversion signal into which the photoacoustic wave signal is converted at the timing of normally receiving the ultrasonic signal in the ultrasonic imager. Consequently, the ultrasonic imager can more easily process the conversion signal into which the photoacoustic wave signal is converted.

In the aforementioned photoacoustic wave signal converter according to the aspect, the signal conversion portion is preferably configured to generate the conversion signal corresponding to a detection time in which the ultrasonic signal is detected when the ultrasonic probe receives the ultrasonic wave by doubling a detection time in which the photoacoustic wave signal is detected when the ultrasonic probe receives a photoacoustic wave and to output the conversion signal that is generated to the ultrasonic imager. According to this structure, the conversion signal obtained by associating the detection time in which the photoacoustic wave signal is detected with the detection time in which the ultrasonic signal is detected can be easily generated. Furthermore, the conversion signal obtained by associating the detection time in which the photoacoustic wave signal is detected with the detection time in which the ultrasonic signal is detected is input into the ultrasonic imager, and hence the ultrasonic imager can easily process the conversion signal into which the photoacoustic wave signal is converted.

In the aforementioned photoacoustic wave signal converter according to the aspect, the signal conversion portion preferably includes a signal processing portion that generates the conversion signal associated with each of a plurality of detection elements of the ultrasonic probe on the basis of the photoacoustic wave signal received by the receiving portion. According to this structure, the conversion signal is divided with respect to each of the detection elements of the ultrasonic probe, and hence the ultrasonic imager can more easily process the conversion signal.

In this case, the receiving portion preferably includes an analog-digital conversion portion that converts the photoacoustic wave signal from an analog signal into a digital signal when receiving the photoacoustic wave signal, and the signal processing portion of the signal conversion portion is preferably configured to generate the conversion signal on the basis of the photoacoustic wave signal as the digital signal converted by the analog-digital conversion portion of the receiving portion. According to this structure, the signal processing portion of the signal conversion portion can process the photoacoustic wave signal as a digital signal. Consequently, conditions for processing the photoacoustic wave signal by the signal processing portion can be easily changed unlike the case where a photoacoustic wave signal as an analog signal is processed.

In the aforementioned structure in which the receiving portion includes the analog-digital conversion portion, the receiving portion preferably further includes an amplification portion that amplifies the photoacoustic wave signal, and the analog-digital conversion portion of the receiving portion is preferably configured to convert the photoacoustic wave signal having been amplified by the amplification portion from an analog signal into a digital signal. According to this structure, even when a faint photoacoustic wave signal is obtained, the amplification portion can amplify the faint photoacoustic wave signal. Consequently, the analog-digital conversion portion can convert the photoacoustic wave signal having been amplified by the amplification portion from an analog signal into a digital signal. Therefore, the bit resolution of the analog-digital conversion portion can be effectively used. Thus, the photoacoustic wave signal as an analog signal can be accurately converted into the photoacoustic wave signal as a digital signal.

In this case, the signal conversion portion preferably further includes a signal adjustment portion that adjusts the amplitude of the conversion signal by converting the conversion signal output from the signal processing portion of the signal conversion portion from a digital signal into an analog signal and multiplying the conversion signal converted into the analog signal by an amplification smaller than 1. According to this structure, even when the signal processing portion generates the conversion signal in a state where the amplification portion amplifies the photoacoustic wave signal, the amplitude of the conversion signal converted into the analog signal can be adjusted to be small before output to the ultrasonic imager. Consequently, output of the conversion signal having an amplitude larger than an amplitude capable of being recognized in the ultrasonic imager to the ultrasonic imager can be significantly reduced or prevented.

In the aforementioned structure in which the receiving portion includes the analog-digital conversion portion and the amplification portion, the receiving portion preferably includes a plurality of analog-digital conversion portions and a plurality of amplification portions equal in number to the plurality of detection elements of the ultrasonic probe. According to this structure, the photoacoustic wave signal detected by each of the plurality of detection elements can be amplified concurrently by the corresponding amplification portion and be converted from an analog signal into a digital signal by the corresponding analog-digital conversion portion. Consequently, the photoacoustic wave signal can be promptly processed, and hence the photoacoustic wave signal can be promptly converted into the conversion signal.

In the aforementioned structure in which the receiving portion includes the analog-digital conversion portion and the amplification portion, the receiving portion preferably includes the analog-digital conversion portion and the amplification portion fewer in number than the plurality of detection elements of the ultrasonic probe, and the photoacoustic wave signal converter preferably further includes a reception switching portion configured to allow the receiving portion to receive photoacoustic wave signals detected by the detection elements of the ultrasonic probe in plural batches. According to this structure, the photoacoustic wave signals detected by the plurality of respective detection elements can be reliably acquired while the structure of the receiving portion is simplified.

The aforementioned photoacoustic wave signal converter according to the aspect preferably further includes a switching portion capable of selectively switching between a first signal path for receiving the photoacoustic wave signal through the receiving portion and outputting the conversion signal to the ultrasonic imager when the ultrasonic probe detects the photoacoustic wave signal and a second signal path for outputting the ultrasonic signal to the ultrasonic imager not through the receiving portion when the ultrasonic probe detects the ultrasonic signal. According to this structure, the switching portion can switch between the first signal path and the second signal path, and hence the case of detecting the photoacoustic wave signal and the case of detecting the ultrasonic signal can be easily switched. Consequently, measurement can be performed by easily switching between detection of the photoacoustic wave signal and detection of the ultrasonic signal according to the situation of measurement by a user.

The aforementioned photoacoustic wave signal converter according to the aspect preferably further includes a light source driving portion configured to control a light source provided outside in order to generate the photoacoustic wave signal to emit pulsed light. According to this structure, the user can perform both conversion of the photoacoustic wave signal and drive control of the light source simply by preparing the photoacoustic wave signal converter. Consequently, measurement of a photoacoustic wave using the ultrasonic imager can be more easily performed as compared with the case where the light source driving portion is provided separately from the photoacoustic wave signal converter.

In this case, the light source driving portion is preferably configured to control the light source including at least one of a light-emitting diode element, a semiconductor laser element, and an organic light-emitting diode element to emit the pulsed light. According to this structure, the light-emitting diode element, the semiconductor laser element, and the organic light-emitting diode element capable of emitting pulsed light by a relatively simple light source driving mechanism are driven by the light source driving portion, and hence an increase in the size of the light source driving portion can be significantly reduced or prevented. Consequently, an increase in the size of the converter photoacoustic wave signal provided with the light source driving portion can be significantly reduced or prevented.

In the aforementioned structure further including the light source driving portion, the light source driving portion is preferably configured to be capable of adjusting a current value for controlling the light source provided outside in order to generate the photoacoustic wave signal to emit the pulsed light on the basis of a control signal from the ultrasonic imager. According to this structure, output of light applied from the light source can be easily adjusted by the ultrasonic imager. Consequently, light output can be easily adjusted according to measurement conditions.

In the aforementioned photoacoustic wave signal converter according to the aspect, the photoacoustic wave signal converter including the receiving portion and the signal conversion portion is preferably arranged between the ultrasonic probe and the ultrasonic imager. According to this structure, the marketed ultrasonic imager is easily allowed to serve also as a photoacoustic imager simply by setting the photoacoustic wave signal converter between the ultrasonic probe and the ultrasonic imager.

In the aforementioned photoacoustic wave signal converter according to the aspect, the photoacoustic wave signal converter including the receiving portion and the signal conversion portion is preferably incorporated in the ultrasonic probe. According to this structure, the photoacoustic wave signal converter is incorporated in the ultrasonic probe, and hence the structure can be further simplified by configuring the photoacoustic wave signal converter as an apparatus integrated with the ultrasonic probe.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a diagram showing the overall structure of an ultrasonic/photoacoustic wave imaging system according to first to third embodiments of the present invention;
Fig. 2 is a block diagram showing the overall structure of the ultrasonic/photoacoustic wave imaging system according to the first embodiment of the present invention;
Fig. 3 is a diagram for illustrating detection of photoacoustic wave signals by detection elements of an ultrasonic probe according to the first embodiment of the present invention;
Fig. 4 is a diagram for illustrating photoacoustic wave frame data in a photoacoustic wave signal converter according to the first embodiment of the present invention;
Fig. 5 is a diagram for illustrating a light-emission cycle of a light source unit and the photoacoustic wave signals acquired by the photoacoustic wave signal converter according to the first embodiment of the present invention;
Fig. 6 is a diagram for illustrating an image based on the photoacoustic wave signals and conversion signals based on this image generated by the photoacoustic wave signal converter according to the first embodiment of the present invention;
Fig. 7 is a diagram for illustrating conversion signal frame data in the photoacoustic wave signal converter according to the first embodiment of the present invention;
Fig. 8 is a diagram for illustrating a transmission cycle of ultrasonic signals of an ultrasonic imager and output of the conversion signals of the photoacoustic wave signal converter according to the first embodiment of the present invention;
Fig. 9 is a flowchart for illustrating photoacoustic wave signal conversion processing performed by the photoacoustic wave signal converter according to the first embodiment of the present invention;
Fig. 10 is a block diagram showing the overall structure of an ultrasonic/photoacoustic wave imaging system according to a second embodiment of the present invention;
Fig. 11 is a flowchart for illustrating photoacoustic wave signal conversion processing performed by a photoacoustic wave signal converter according to the second embodiment of the present invention;
Fig. 12 is a block diagram showing the overall structure of an ultrasonic/photoacoustic wave imaging system according to a third embodiment of the present invention;
Fig. 13 is a flowchart for illustrating photoacoustic wave signal conversion processing performed by a photoacoustic wave signal converter according to the third embodiment of the present invention; and
Fig. 14 is a block diagram showing the overall structure of an ultrasonic/photoacoustic wave imaging system according to a fourth embodiment of the present invention.

### DESCRIPTION OF THE PREFERRED EMBODIMENTS

Embodiments of the present invention are hereinafter described with reference to the drawings.

### (First Embodiment)

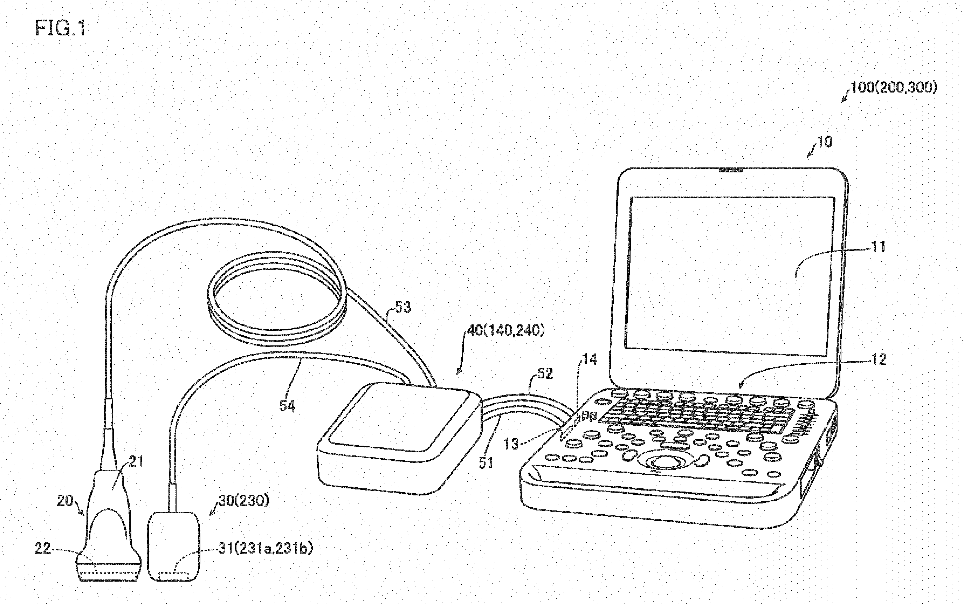

The structure of an ultrasonic/photoacoustic wave imaging system 100 according to a first embodiment of the present invention is now described with reference to Figs. 1 to 8.

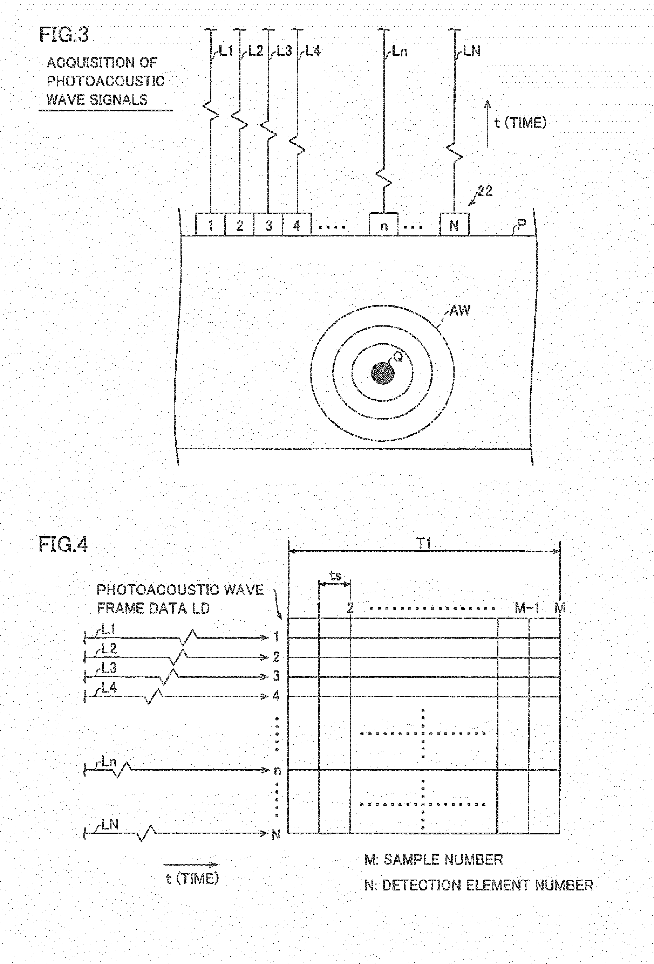

The ultrasonic/photoacoustic wave imaging system 100 according to the first embodiment of the present invention includes an ultrasonic imager 10, an ultrasonic probe 20, a light source unit 30, and a photoacoustic wave signal converter (hereinafter referred to as the converter) 40, as shown in Fig. 1. In the ultrasonic/photoacoustic wave imaging system 100, the ultrasonic imager 10 is connected to the ultrasonic probe 20 and the light source unit 30 through the converter 40. As the ultrasonic imager 10 and the ultrasonic probe 20, generally marketed instruments employed to image the inside of a specimen P (see Fig. 3) by ultrasonic waves can be employed.

In the ultrasonic/photoacoustic wave imaging system 100, the ultrasonic probe 20 is configured to detect received signals (ultrasonic signals) of ultrasonic waves reflected in the specimen P and received signals (photoacoustic wave signals) of a photoacoustic wave AW (see Fig. 3) generated in the specimen P. The ultrasonic/photoacoustic wave imaging system 100 is configured to be capable of generating both an ultrasonic image based on the ultrasonic signals and a photoacoustic wave image based on the photoacoustic wave signals by the ultrasonic imager 10.

Specifically, light is first applied to the specimen P by the light source unit 30 when the photoacoustic wave image is generated. Then, a photoacoustic wave AW (see Fig. 3) generated from a detection object Q (see Fig. 3) in the specimen P that has absorbed the light is received by the ultrasonic probe 20, whereby photoacoustic wave signals are detected. Thereafter, the detected photoacoustic wave signals are converted into conversion signals corresponding to ultrasonic signals by the converter 40 and are input into the ultrasonic imager 10. Consequently, the ultrasonic imager 10 can generate the photoacoustic wave image on the basis of the conversion signals by processing similar to normal ultrasonic image generation processing. Signal conversion from the photoacoustic wave signals to the conversion signals is described later in detail.

When the ultrasonic image is generated, on the other hand, ultrasonic waves are first applied to the specimen P (see Fig. 3) such as a human body by the ultrasonic probe 20. Then, the ultrasonic waves reflected in the specimen P are received by the ultrasonic probe 20, whereby ultrasonic signals are detected. Thereafter, the detected ultrasonic signals are input into the ultrasonic imager 10 through the converter 40. At this time, the ultrasonic signals detected by the ultrasonic probe 20 are directly input into the ultrasonic imager 10 without conversion in the converter 40. Consequently, the ultrasonic imager 10 can generate the ultrasonic image, similarly to the case where the ultrasonic imager 10 and the ultrasonic probe 20 are directly connected to each other in order to generate an ultrasonic image.

The ultrasonic/photoacoustic wave imaging system 100 is configured to be capable of generating a synthetic image obtained by superimposing the generated ultrasonic image and the generated photoacoustic wave image. Thus, the ultrasonic/photoacoustic wave imaging system 100 is configured to visualize a variety of information in the specimen P. Each structural requirement of the ultrasonic/photoacoustic wave imaging system 100 is now described in detail.

As shown in Fig. 1, the ultrasonic imager 10 is provided with a monitor 11 and an operation portion 12.

The monitor 11 includes a common liquid crystal monitor and is configured to be capable of displaying an ultrasonic image, a photoacoustic wave image, a synthetic image, various operation screens, etc.

The operation portion 12 includes various adjustment buttons, a keyboard, etc. and is arranged on the display surface side of the monitor 11. A user can operate the ultrasonic imager 10 by the operation portion 12 while visually recognizing an image displayed on the monitor 11.

The ultrasonic imager 10 is provided with a probe connection portion 13 and a signal output portion 14 on a side surface of the operation portion 12. The probe connection portion 13 is generally a terminal connected with the ultrasonic probe 20. According to the first embodiment, the probe connection portion 13 is connected with the converter 40 through a wire 51. The probe connection portion 13 is configured to input and output ultrasonic signals as transmitted signals of ultrasonic waves and ultrasonic signals as received signals of ultrasonic waves and to input photoacoustic wave signals. The signal output portion 14 is a terminal under prescribed standards such as the USB (Universal Serial Bus) standards. The signal output portion 14 is connected with the converter 40 through a wire 52. The signal output portion 14 is configured to output a control signal from the ultrasonic imager 10 to the converter 40.

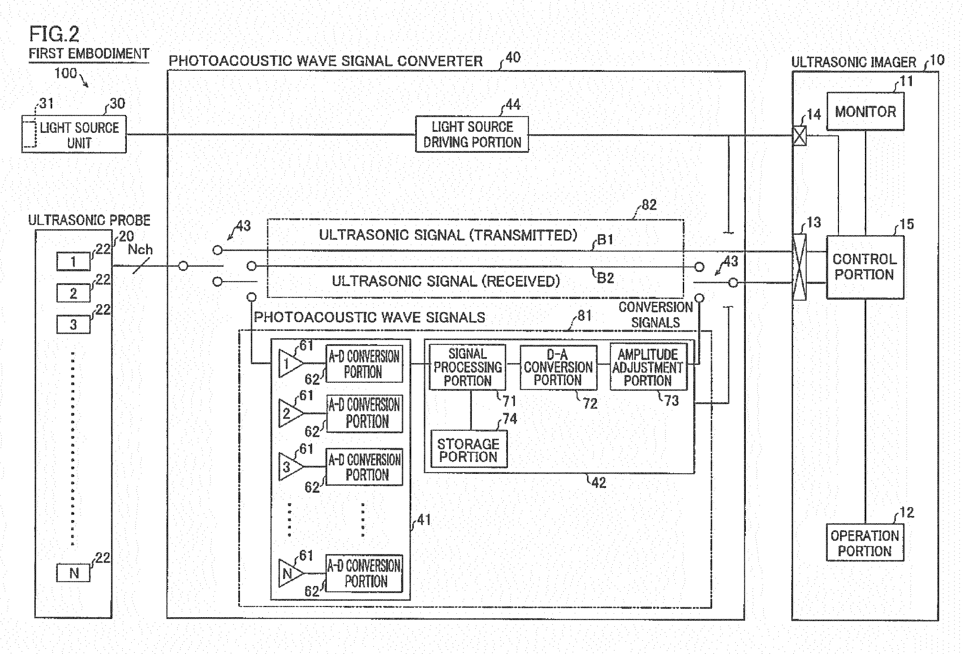

As shown in Fig. 2, the ultrasonic imager 10 is provided with a control portion 15. The control portion 15 includes a CPU and is configured to control each component of the ultrasonic imager 10. The control portion 15 is configured to perform control of generating an ultrasonic image and a photoacoustic wave image on the basis of the ultrasonic signals detected by the ultrasonic probe 20 and the conversion signals detected by the ultrasonic probe 20, obtained by conversion by the converter 40, respectively. The control portion 15 is configured to perform control of generating a synthetic image obtained by superimposing an ultrasonic image and a photoacoustic wave image. The control portion 15 is configured to perform control of displaying an image such as an ultrasonic image, a photoacoustic wave image, a synthetic image, or the like on the monitor 11. The control portion 15 is configured to be capable of outputting various control portions to the converter 40 through the signal output portion 14 according to user's operation of the operation portion 12.

As shown in Figs. 1 and 2, the ultrasonic probe 20 is a probe configured to transmit and receive ultrasonic waves. The ultrasonic probe 20 is connected to the converter 40 through a wire 53. The ultrasonic probe 20 is configured such that a signal is input thereinto from the converter 40 and output therefrom to the converter 40 through the wire 53. The ultrasonic probe 20 is configured such that a signal is input thereinto from the ultrasonic imager 10 and output therefrom to the ultrasonic imager 10 through the converter 40.

The ultrasonic probe 20 includes a housing 21 and a plurality of detection elements 22. The plurality of detection elements 22 include piezoelectric elements and are arranged in an array in the vicinity of a tip end of an internal portion of the housing 21. According to the first embodiment, N (also referred to as N channels) detection elements 22 are provided. The number N of the detection elements 22 can be 64, 128, 192, or 256, for example.

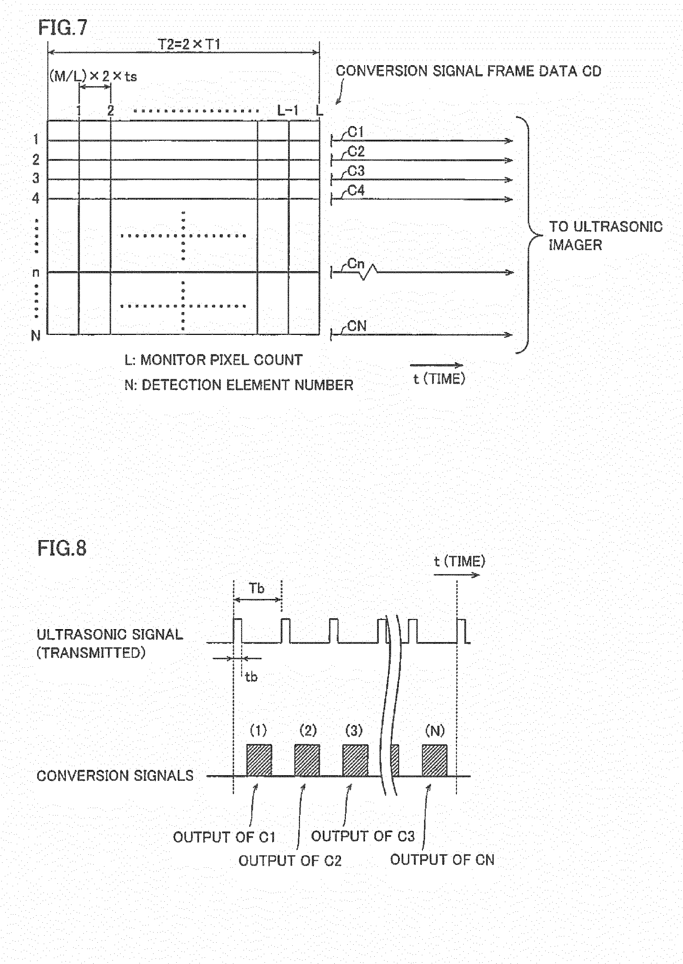

The ultrasonic probe 20 is configured to be capable of generating ultrasonic waves by vibrating the detection elements 22 on the basis of ultrasonic signals as transmitted signals from the control portion 15 of the ultrasonic imager 10. Specifically, the ultrasonic probe 20 is configured to repetitively generate a pulse ultrasound having a pulse width tb (see Fig. 8) in a transmission cycle Tb (see Fig. 8) on the basis of the ultrasonic signals as the transmitted signals. Furthermore, the ultrasonic probe 20 is configured to detect ultrasonic signals as received signals corresponding to respective pulse ultrasounds by vibration of the detection elements 22 by the ultrasonic waves reflected in the specimen P. The ultrasonic probe 20 is configured to detect photoacoustic wave signals by vibration of the detection elements 22 by the photoacoustic wave AW generated from the detection object Q in the specimen P that has absorbed the light applied from the light source unit 30. The ultrasonic probe 20 is configured to output the detected ultrasonic signals and photoacoustic wave signals to the converter 40 through the wire 53.

As shown in Figs. 1 and 2, the light source unit 30 is connected to the converter 40 through a wire 54. The light source unit 30 is a unit configured to apply light to the specimen P. During measurement of a photoacoustic wave AW, the light source unit 30 is employed in contact with a surface of the specimen P together with the ultrasonic probe 20.

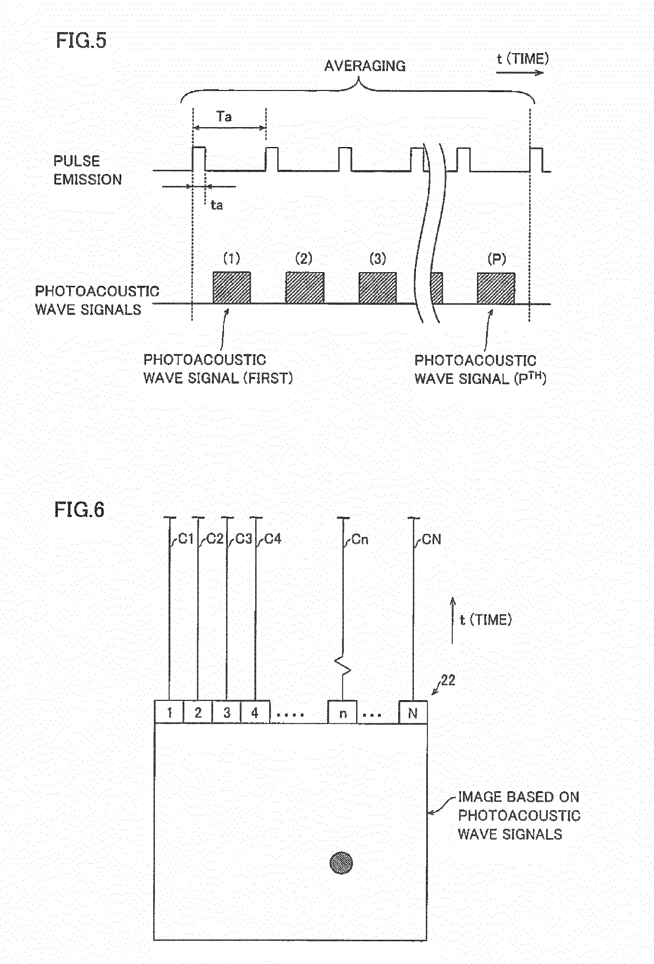

The light source unit 30 includes a light source 31 and is configured to apply light for measurement from the light source 31 to the specimen P. The light source 31 of the light source unit 30 is configured to repetitively emit pulsed light having a pulse width ta (see Fig. 5) in a light-emission cycle Ta (see Fig. 5) on the basis of a control signal from a light source driving portion 44, described later, of the converter 40. During a prescribed period after pulsed light emission and before subsequent pulsed light emission, photoacoustic wave signals are repetitively acquired by the converter 40.

The light source 31 is configured to generate light (light with a center wavelength at about 700 nm to about 1000 nm, for example) with a measurement wavelength of an infrared region suitable for measuring the specimen P such as a human body. As the light source 31, a semiconductor light-emitting element such as a light-emitting diode element, a semiconductor laser element, or an organic light-emitting diode element can be employed, for example. The measurement wavelength of the light source 31 may be properly determined according to the detection object Q desired to be detected.

According to the first embodiment, the converter 40 is provided between the ultrasonic imager 10 and both the ultrasonic probe 20 and the light source unit 30, as shown in Fig. 1. The converter 40 is a device that converts photoacoustic wave signals detected by the ultrasonic probe 20 into signals (conversion signals) capable of being processed by the ultrasonic imager 10. The structure of the converter 40 is now described in detail.

The converter 40 includes a receiving portion 41 and a signal conversion portion 42. The converter 40 is configured to receive photoacoustic wave signals by the receiving portion 41, to convert the received photoacoustic wave signals into conversion signals by the signal conversion portion 42, and to output the conversion signals to the ultrasonic imager 10.

The receiving portion 41 is provided with a plurality of (N) amplification portions 61 and a plurality of (N) analog-digital conversion portions (hereinafter referred to as the A-D conversion portions) 62 in correspondence to the plurality of (N) detection elements 22 of the ultrasonic probe 20. In other words, the receiving portion 41 is configured to receive a photoacoustic wave signal detected by a first detection element 22 through a first amplification portion 61 and a first A-D conversion portion 62 associated therewith. The receiving portion 41 is configured to receive photoacoustic wave signals through correspondingly numbered amplification portions 61 and A-D conversion portions 62, similarly up to an N^{th} detection element 22. In Fig. 2, a connection state between each detection element 22 and each amplification portion 61 is simplified for ease of illustration.

The amplification portions 61 are configured to amplify (about 300 times to about 30,000 times, for example) photoacoustic wave signals and to output the photoacoustic wave signals to the A-D conversion portions 62.

The A-D conversion portions 62 are configured to convert the photoacoustic wave signals having been amplified by the amplification portions 61 from analog signals into digital signals with a prescribed sampling frequency and prescribed bit resolution. In general, the photoacoustic wave signals are often faint. Therefore, as described above, the bit resolution of the A-D conversion portions 62 can be effectively used by the A-D conversion of the photoacoustic wave signals having been amplified by the amplification portions 61. The A-D conversion portions 62 are configured to output the photoacoustic wave signals as digital signals to the signal conversion portion 42.

The signal conversion portion 42 includes a signal processing portion 71, a digital-analog conversion portion (hereinafter referred to as the D-A conversion portion) 72, an amplitude adjustment portion 73, and a storage portion 74. Both the D-A conversion portion 72 and the amplitude adjustment portion 73 are examples of the "signal adjustment portion" in the present invention. The storage portion 74 includes a ROM, a RAM, etc. and is configured to store various data and programs etc. employed by the signal processing portion 71.

According to the first embodiment, the signal processing portion 71 includes an FPGA (Field Programmable Gate Array) and is configured to generate conversion signals corresponding to ultrasonic signals generated when the ultrasonic probe 20 receives ultrasonic waves on the basis of the photoacoustic wave signals received by the receiving portion 41. In other words, the signal processing portion 71 is configured to convert the photoacoustic wave signals received by the receiving portion 41 into the conversion signals corresponding to the ultrasonic signals. Schematically, the conversion of the photoacoustic wave signals into the conversion signals by the signal processing portion 71 is performed by generation of an image (image data) based on the photoacoustic wave signals and generation of the conversion signals based on the generated image (image data). The conversion of the photoacoustic wave signals into the conversion signals is hereinafter described in detail with reference to Figs. 3 to 8.

The generation of an image based on the photoacoustic wave signals is now described. When the light source unit 30 (see Fig. 2) applies pulsed light to the specimen P, a photoacoustic wave AW is generated from the detection object Q in the specimen P, as shown in Fig. 3. At this time, the photoacoustic wave AW is generated from a wide range at a time by light application. In Fig. 3, only one detection object Q is illustrated for ease of understanding.

Then, the ultrasonic probe 20 (see Fig. 2) receives the photoacoustic wave AW generated from the detection object Q and detects the photoacoustic wave signals by the N respective detection elements 22. In Fig. 3, the photoacoustic wave signals detected by the detection elements 22 are shown as photoacoustic wave signals L1 to LN. The photoacoustic wave signals L1 to LN detected by the detection elements 22 are output from the ultrasonic probe 20 to the converter 40 and are received by the receiving portion 41 of the converter 40. In the receiving portion 41, the photoacoustic wave signals L1 to LN as analog signals are amplified and converted into photoacoustic wave signals L1 to LN as digital signals by the amplification portions 61 and the A-D conversion portions 62 associated with the respective detection elements 22. Then, the photoacoustic wave signals L1 to LN converted into the digital signals are stored in the storage portion 74 of the signal conversion portion 42.

In the signal conversion portion 42, the signal processing portion 71 constructs photoacoustic wave frame data LD on the basis of the photoacoustic wave signals L1 to LN associated with the respective detection elements 22, as shown in Fig. 4. The photoacoustic wave frame data LD is data obtained by configuring information about the width direction of the ultrasonic probe 20 and information about a depth direction from the surface of the specimen P in a matrix. Specifically, the photoacoustic wave frame data LD is configured by a matrix of the number N of detection elements 22 (the detection element number N) and the sample number M. The sample number M is a sample number for a signal up to a depth desired to be imaged in each of the photoacoustic wave signals L1 to LN. When a depth desired to be imaged is 6 cm (0.06 m) from the surface of the specimen P, the sound velocity in a human body is 1,500 (m/s), and the prescribed sampling frequency of the A-D conversion portions 62 is 20 x 10⁶ Hz, for example, the sample number M is obtained by the following formula: M = (0.06/1500) x 20 x 10⁶ = 800. The sample number M indicates the number of pixels in the depth direction, and in the case of the aforementioned calculation example, for example, there are 800 pixels in the depth direction.

The sample number M corresponds to the detection time T1 of each of the photoacoustic wave signals L1 to LN detected by the ultrasonic probe 20. When a time interval (sampling time) between points of the sample number M is ts and the prescribed sampling frequency of the A-D conversion portions 62 is 20 x 10⁶ Hz, for example, the sampling time ts is 0.05 µs. In this case, the detection time T1 of each of the photoacoustic wave signals L1 to LN is 0.05 µs x 800 = 40 µs obtained by multiplying the sampling time ts by the sample number M = 800. One piece of photoacoustic wave frame data LD shown in Fig. 4 is constructed per pulse emission by the light source 31 of the light source unit 30.

As shown in Fig. 5, the signal processing portion 71 constructs a plurality of (P) pieces of photoacoustic wave frame data LD corresponding to respective photoacoustic wave signals received due to pulsed light emitted a plurality of (P) times. The signal processing portion 71 is configured to average the plurality of (P) pieces of constructed photoacoustic wave frame data LD. Furthermore, the signal processing portion 71 is configured to generate an image (image data) based on the photoacoustic wave signals by an analytical method such as phasing addition on the basis of the averaged frame data LD. Thus, an image (image data) based on the photoacoustic wave signals can be generated while the S/N ratio (signal/noise ratio) of the photoacoustic wave signals is improved by averaging, and hence an image that accurately reflects a state inside the specimen P can be generated.

The generation of the conversion signals based on the generated image is now described. As shown in Fig. 6, the signal processing portion 71 is configured to generate conversion signals C1 to CN corresponding to ultrasonic signals generated when the ultrasonic probe 20 receives ultrasonic waves on the basis of the image based on the photoacoustic wave signals. More specifically, the signal processing portion 71 is configured to generate the conversion signals C1 to CN corresponding to the ultrasonic signals generated when the respective detection elements 22 of the ultrasonic probe 20 sequentially transmit ultrasonic waves and sequentially receive the ultrasonic waves reflected in the specimen P on the basis of the image based on the photoacoustic wave signals. In this case, the signal processing portion 71 is configured to generate the conversion signals C1 to CN in correspondence to the detection times (times T2 described later) in which the ultrasonic signals are detected when the ultrasonic probe 20 receives the ultrasonic waves by substantially doubling the detection times T1 in which the photoacoustic wave signals L1 to LN are detected when the ultrasonic probe 20 receives the photoacoustic wave AW. In other words, the signal processing portion 71 is configured to generate the conversion signals C1 to CN associated with the plurality of (N) respective detection elements 22 of the ultrasonic probe 20 on the basis of the image based on the photoacoustic wave signals. Thus, the photoacoustic wave signals detected in parallel (simultaneously) by the N detection elements 22 of the ultrasonic probe 20 are converted into the conversion signals C1 to CN corresponding to the ultrasonic signals.

The signal processing portion 71 is configured to generate conversion signal frame data CD containing the conversion signals C1 to CN on the basis of the image based on the photoacoustic wave signals at this time, as shown in Fig. 7.

The conversion signal frame data CD is data obtained by configuring the information about the width direction of the ultrasonic probe 20 and the information about the depth direction from the surface of the specimen P in a matrix, similarly to the photoacoustic wave frame data LD. The conversion signal frame data CD is configured by a matrix of the detection element number N and the number of pixels (monitor pixel count) L of the monitor 11 of the ultrasonic imager 10 in the depth direction. The sample number M of the photoacoustic wave frame data LD is equal to or more than the monitor pixel count L of the conversion signal frame data CD. The sample number M can be about twice to about five times the monitor pixel count L, for example. Thus, data (conversion signals C1 to CN) can be output in a state where the data is compressed according to the number of pixels (monitor pixel count L) capable of being displayed on the ultrasonic imager 10 when the data is output to the ultrasonic imager 10 while an accurate image that accurately reflects the state inside the specimen P with the sample number M larger than the monitor pixel count L is generated when the image based on the photoacoustic wave signals is generated.

In the conversion signal frame data CD, a time corresponding to the monitor pixel count L has the time T2 (= 2 x T1) twice the detection time T1 in the photoacoustic wave frame data LD. In other words, in the conversion signal frame data CD, the conversion signals C1 to CN each having a time obtained by doubling the detection time T1 of each of the photoacoustic wave signals L1 to LN detected by the ultrasonic probe 20 are generated. Comparing the cases where the ultrasonic signals and the photoacoustic wave signals are detected with respect to the detection object Q at substantially the same position, the detection times substantially double in the case where the ultrasonic signals are detected, as compared with the case where the photoacoustic wave signals are detected. Therefore, the conversion signals C1 to CN each having the time obtained by doubling the detection time T1 of each of the photoacoustic wave signals L1 to LN, whereby the conversion signals C1 to CN corresponding to the detection times (i.e. times T2) in which the ultrasonic signals are detected are generated when the ultrasonic probe 20 receives the ultrasonic waves. Consequently, a distance of the specimen P in the depth direction can be accurately acquired (calculated) from the detection times of the conversion signals C1 to CN when a photoacoustic wave image is generated in the ultrasonic imager 10. In this case, a time interval between points of the monitor pixel count L can be expressed in (M/L) x 2 x ts. When the sample number M and the monitor pixel count L have the same value, for example, the time interval between the points of the monitor pixel count L is expressed in 2 x ts. When the sample number M is a value twice the monitor pixel count L, the time interval between the points of the monitor pixel count L is expressed in 2 x 2 x ts.

As shown in Fig. 8, the signal processing portion 71 is configured to sequentially output the conversion signals C1 to CN associated with the plurality of (N) respective detection elements 22 of the ultrasonic probe 20 to the ultrasonic imager 10 according to a reception cycle (i.e. the transmission cycle Tb) of the ultrasonic signals of the ultrasonic imager 10. The signal processing portion 71 is configured to output the conversion signals C1 to CN to the ultrasonic imager 10 through the D-A conversion portion 72 and the amplitude adjustment portion 73 at this time.

The D-A conversion portion 72 is configured to convert the conversion signals C1 to CN sequentially output from the signal processing portion 71 from digital signals into analog signals and to output the conversion signals C1 to CN to the amplitude adjustment portion 73.

The amplitude adjustment portion 73 includes a VGA (Variable Gain Amplifier) and is configured to adjust the amplitude of the conversion signals C1 to CN by multiplying the conversion signals C1 to CN converted into the analog signals by the D-A conversion portion 72 by an amplification smaller than 1. Then, the conversion signals C1 to CN whose amplitude has been adjusted by the amplitude adjustment portion 73 are sequentially output to the ultrasonic imager 10. Consequently, signals (conversion signals C1 to CN) capable of being processed by the ultrasonic imager 10 are input into the ultrasonic imager 10 at the timing of normally receiving the ultrasonic signals. Therefore, the ultrasonic imager 10 can generate a photoacoustic wave image on the basis of the conversion signals C1 to CN, similarly to an ultrasonic image based on the ultrasonic signals.

Conditions for conversion of the photoacoustic wave signals into the conversion signals by the signal conversion portion 42 can be changed on the basis of a control signal from the control portion 15 of the ultrasonic imager 10. For example, conditions for imaging such as the sample number M used to generate the image based on the photoacoustic wave signals can be changed.

According to the first embodiment, the converter 40 is provided with a first signal path 81 as a signal path in the case where the ultrasonic probe 20 detects the photoacoustic wave signals (in a photoacoustic wave detection mode) and a second signal path 82 as a signal path in the case where the ultrasonic probe 20 detects the ultrasonic signals (in ultrasonic wave detection mode), as shown in Fig. 2.

The first signal path 81 is a signal path including the receiving portion 41 and the signal conversion portion 42. In other words, the first signal path 81 is a signal path for outputting signals to the ultrasonic imager 10 while the receiving portion 41 receives the photoacoustic wave signals and the signal conversion portion 42 converts the received photoacoustic wave signals into the conversion signals.

The second signal path 82 is a signal path including a signal line B1 and a signal line B2 that connect the ultrasonic probe 20 and the ultrasonic imager 10 to each other not through the receiving portion 41. The signal line B1 is a signal line for outputting the ultrasonic signals as the transmitted signals of the ultrasonic waves generated by the ultrasonic imager 10 to the ultrasonic probe 20. The signal line B2 is a signal line for outputting the ultrasonic signals as the received signals of the ultrasonic waves received by the ultrasonic probe 20 to the ultrasonic imager 10. In other words, the second signal path 82 is a signal path for directly transferring the ultrasonic signals as the transmitted signals and the received signals by the ultrasonic probe 20 and the ultrasonic imager 10 not through the receiving portion 41.

According to the first embodiment, the converter 40 is provided with a plurality of (two) switching portions 43 configured to selectively switch between the first signal path 81 and the second signal path 82. Specifically, the plurality of switching portions 43 are configured to selectively switch to the first signal path 81 in the photoacoustic wave detection mode and to selectively switch to the second signal path 82 in the ultrasonic wave detection mode on the basis of a control signal from the control portion 15 of the ultrasonic imager 10. In the ultrasonic/photoacoustic wave imaging system 100, the switching portions 43 of the converter 40 switch between the first signal path 81 and the second signal path 82 when the photoacoustic wave detection mode and the ultrasonic wave detection mode are switched according to user's operation of the operation portion 12 of the ultrasonic imager 10, for example.

According to the first embodiment, the converter 40 is provided with the light source driving portion 44. The light source driving portion 44 is configured to control the light source 31 of the light source unit 30 provided outside to emit pulsed light. Specifically, the light source driving portion 44 is configured to control the light source 31 of the light source unit 30 to repetitively emit pulsed light having a pulse width ta in the light-emission cycle Ta. The light source driving portion 44 is configured to be capable of adjusting the pulse width ta, the light-emission cycle Ta, and a current value for driving the light source 31 on the basis of a control signal from the control portion 15 of the ultrasonic imager 10. In other words, in the ultrasonic/photoacoustic wave imaging system 100, the converter 40 is configured to be capable of changing light-application conditions for the light source unit 30 by changing the setting of the light source driving portion 44 through the ultrasonic imager 10.

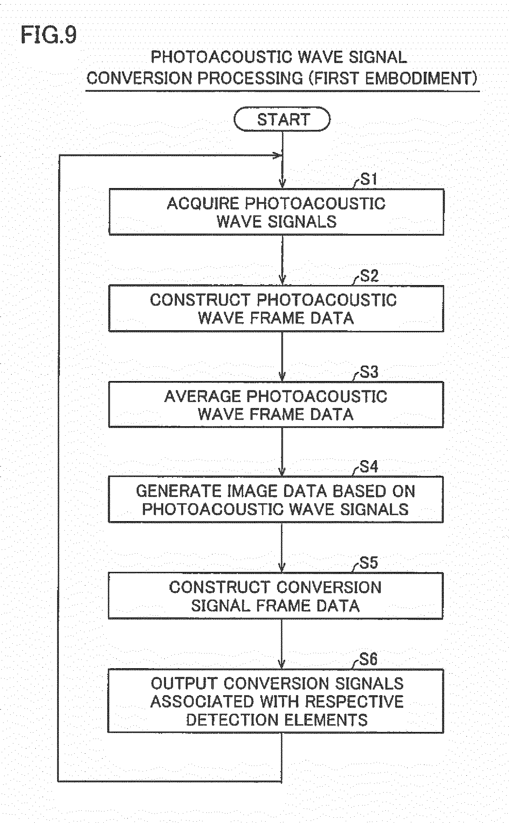

Photoacoustic wave signal conversion processing performed by the signal processing portion 71 of the converter 40 is now described on the basis of a flowchart with reference to Fig. 9.

First, the signal processing portion 71 acquires photoacoustic wave signals at a step S1. Specifically, the signal processing portion 71 acquires the photoacoustic wave signals L1 to LN (see Fig. 4) received by the receiving portion 41 (see Fig. 2) and stored in the storage portion 74 (see Fig. 2).

Then, the signal processing portion 71 constructs the photoacoustic wave frame data LD (see Fig. 4) at a step S2. Specifically, the signal processing portion 71 constructs the photoacoustic wave frame data LD on the basis of the photoacoustic wave signals L1 to LN acquired at the step S1. Furthermore, the signal processing portion 71 constructs P pieces of photoacoustic wave frame data LD corresponding to the number of times of addition in averaging at the step S2.

Then, the signal processing portion 71 averages a plurality of (P) pieces of photoacoustic wave frame data LD at a step S3.

Then, the signal processing portion 71 generates an image (image data) of the inside of the specimen P on the basis of the averaged photoacoustic wave frame data LD at a step S4. In other words, the signal processing portion 71 generates an image (image data) of the inside of the specimen P based on the photoacoustic wave signals received by the receiving portion 41 at the step S4.

Then, the signal processing portion 71 constructs the conversion signal frame data DC (see Fig. 7) at a step S5. Specifically, at the step S5, the signal processing portion 71 constructs the conversion signal frame data DC containing the conversion signals C1 to CN associated with the respective detection elements 22 (see Fig. 2) on the basis of the image based on the photoacoustic wave signals generated at the step S4.

Then, the signal processing portion 71 sequentially outputs the conversion signals C1 to CN associated with the respective detection elements 22 (see Fig. 2) to the ultrasonic imager 10 according to the reception cycle of the ultrasonic signals at a step S6. Consequently, a photoacoustic wave image is generated in the ultrasonic imager 10 (see Fig. 2), and the generated photoacoustic wave image is displayed on the monitor 11 (see Fig. 2). Then, the signal processing portion 71 returns to the step S1 and acquires subsequent photoacoustic wave signals.

According to the first embodiment, the following effects can be obtained.

According to the first embodiment, as hereinabove described, the converter 40 is provided with the signal conversion portion 42 that generates the conversion signals corresponding to the ultrasonic signals generated when the ultrasonic probe 20 receives the ultrasonic waves on the basis of the photoacoustic wave signals received by the receiving portion 41 and outputs the generated conversion signals to the ultrasonic imager 10. Thus, even if the ultrasonic probe 20 captures the received signals (photoacoustic wave signals) of the photoacoustic waves in parallel (simultaneously), the captured photoacoustic wave signals can be converted into the conversion signals corresponding to the ultrasonic signals generated when the ultrasonic probe 20 receives the ultrasonic waves. Consequently, the conversion signals corresponding to the ultrasonic signals can be output to the ultrasonic imager 10, and hence the ultrasonic imager 10 can process the conversion signals into which the photoacoustic wave signals are converted, substantially similarly to the case of processing the ultrasonic signals. Therefore, the marketed ultrasonic imager 10 can serve as a photoacoustic imager and display a photoacoustic wave image, using the converter 40 without changing imager body hardware.

According to the first embodiment, as hereinabove described, the signal conversion portion 42 is configured to generate the conversion signals on the basis of the photoacoustic wave signal received by the receiving portion 41 and to output the generated conversion signals to the ultrasonic imager 10 according to the reception cycle (i.e. the transmission cycle Tb) of the ultrasonic signals received by the ultrasonic imager 10. Thus, the ultrasonic imager 10 can receive the conversion signals into which the photoacoustic wave signals are converted at the timing of normally receiving the ultrasonic signals in the ultrasonic imager 10. Consequently, the ultrasonic imager 10 can more easily process the conversion signals into which the photoacoustic wave signals are converted.

According to the first embodiment, as hereinabove described, the signal conversion portion 42 is configured to generate the conversion signals corresponding to the detection times (i.e. times T2) in which the ultrasonic signals are detected when the ultrasonic probe 20 receives the ultrasonic waves by substantially doubling the detection times T1 in which the photoacoustic wave signals are detected when the ultrasonic probe 20 receives the photoacoustic wave and to output the generated conversion signals to the ultrasonic imager 10. Thus, the conversion signals obtained by associating the detection times T1 in which the photoacoustic wave signals are detected with the detection times (T2) in which the ultrasonic signals are detected can be easily generated. Furthermore, the conversion signals obtained by associating the detection times T1 in which the photoacoustic wave signals are detected with the detection times (T2) in which the ultrasonic signals are detected are input into the ultrasonic imager 10, and hence the ultrasonic imager 10 can easily process the conversion signals into which the photoacoustic wave signals are converted.

According to the first embodiment, as hereinabove described, the signal conversion portion 42 is provided with the signal processing portion 71 that generates the conversion signals associated with the respective detection elements 22 of the ultrasonic probe 20 on the basis of the photoacoustic wave signals received by the receiving portion 41. Thus, the conversion signals are divided with respect to the respective detection elements 22 of the ultrasonic probe 20, and hence the ultrasonic imager 10 can more easily process the conversion signals.

According to the first embodiment, as hereinabove described, the receiving portion 41 is provided with the A-D conversion portions 62 that convert the photoacoustic wave signals from analog signals into digital signals when receiving the photoacoustic wave signals. Furthermore, the signal processing portion 71 of the signal conversion portion 42 is configured to generate the conversion signals on the basis of the photoacoustic wave signals as the digital signals converted by the A-D conversion portions 62 of the receiving portion 41. Thus, the signal processing portion 71 of the signal conversion portion 42 can process the photoacoustic wave signals as digital signals. Consequently, conditions for processing the photoacoustic wave signals by the signal processing portion 71 can be easily changed unlike the case where photoacoustic wave signals as analog signals are processed.

According to the first embodiment, as hereinabove described, the A-D conversion portions 62 of the receiving portion 41 are configured to convert the photoacoustic wave signals having been amplified by the amplification portions 61 from analog signals into digital signals. Thus, even when faint photoacoustic wave signals are obtained, the amplification portions 61 can amplify the faint photoacoustic wave signals. Consequently, the A-D conversion portions 62 can convert the photoacoustic wave signals having been amplified by the amplification portions 61 from analog signals into digital signals. Therefore, the bit resolution of the A-D conversion portions 62 can be effectively used. Thus, the photoacoustic wave signals as analog signals can be accurately converted into the photoacoustic wave signals as digital signals.

According to the first embodiment, as hereinabove described, the signal conversion portion 42 is provided with the D-A conversion portion 72 that converts the conversion signals output from the signal processing portion 71 of the signal conversion portion 42 from digital signals into analog signals and the amplitude adjustment portion 73 that adjusts the amplitude of the conversion signals by multiplying the conversion signals converted into the analog signals by the amplification smaller than 1. Thus, even when the signal processing portion 71 generates the conversion signals in a state where the amplification portions 61 amplify the photoacoustic wave signals, the amplitude of the conversion signals converted into the analog signals can be adjusted to be small before output to the ultrasonic imager 10. Consequently, output of the conversion signals having an amplitude larger than an amplitude capable of being recognized in the ultrasonic imager 10 to the ultrasonic imager 10 can be significantly reduced or prevented.

According to the first embodiment, as hereinabove described, the receiving portion 41 is provided with the plurality of (N) A-D conversion portions 62 and the plurality of (N) amplification portions 61 equal in number to the plurality of (N) detection elements 22 of the ultrasonic probe 20. Thus, the photoacoustic wave signals detected by the plurality of (N) respective detection elements 22 can be amplified concurrently by the corresponding amplification portions 61 and be converted from analog signals into digital signals by the corresponding A-D conversion portions 62. Consequently, the photoacoustic wave signals can be promptly processed, and hence the photoacoustic wave signals can be promptly converted into the conversion signals.

According to the first embodiment, as hereinabove described, the converter 40 is provided with the switching portions 43 capable of selectively switching between the first signal path 81 for receiving the photoacoustic wave signals through the receiving portion 41 and outputting the conversion signals to the ultrasonic imager 10 when the ultrasonic probe 20 detects the photoacoustic wave signals and the second signal path 82 for outputting the ultrasonic signals to the ultrasonic imager 10 not through the receiving portion 41 when the ultrasonic probe 20 detects the ultrasonic signals. Thus, the switching portions 43 can switch between the first signal path 81 and the second signal path 82, and hence the case of detecting the photoacoustic wave signals and the case of detecting the ultrasonic signals can be easily switched. Consequently, measurement can be performed by easily switching between detection of the photoacoustic wave signals and detection of the ultrasonic signals according to the situation of measurement by the user.

According to the first embodiment, as hereinabove described, the converter 40 is provided with the light source driving portion 44 configured to control the light source 31 of the light source unit 30 provided outside in order to generate the photoacoustic wave signals to emit pulsed light. Thus, the user can perform both conversion of the photoacoustic wave signals and drive control of the light source 31 of the light source unit 30 simply by preparing the converter 40. Consequently, measurement of a photoacoustic wave using the ultrasonic imager 10 can be more easily performed as compared with the case where the light source driving portion that drives the light source unit 30 is provided separately from the converter 40.

According to the first embodiment, as hereinabove described, the light source driving portion 44 is configured to control the light source 31 including at least one of a light-emitting diode element, a semiconductor laser element, and an organic light-emitting diode element to emit pulsed light. Thus, the light-emitting diode element, the semiconductor laser element, and the organic light-emitting diode element capable of emitting pulsed light by a relatively simple light source driving mechanism are driven by the light source driving portion 44, and hence an increase in the size of the light source driving portion 44 can be significantly reduced or prevented. Consequently, an increase in the size of the converter 40 provided with the light source driving portion 44 can be significantly reduced or prevented.

According to the first embodiment, as hereinabove described, the light source driving portion 44 is configured to be capable of adjusting the current value for controlling the light source 31 provided outside in order to generate the photoacoustic wave signals to emit pulsed light on the basis of the control signal from the ultrasonic imager 10. Thus, output of light applied from the light source 31 can be easily adjusted by the ultrasonic imager 10. Consequently, light output can be easily adjusted according to measurement conditions.

According to the first embodiment, as hereinabove described, the converter 40 including the receiving portion 41 and the signal conversion portion 42 is arranged between the ultrasonic probe 20 and the ultrasonic imager 10. Thus, the marketed ultrasonic imager 10 is easily allowed to serve also as a photoacoustic imager simply by setting the converter 40 between the ultrasonic probe 20 and the ultrasonic imager 10.

### (Second Embodiment)

A second embodiment is now described with reference to Figs. 1, 10, and 11. In this second embodiment, amplification portions 161 and A-D conversion portions 162 fewer in number than N detection elements 22 are provided unlike the aforementioned first embodiment in which the N amplification portions 61 and the N A-D conversion portions 62 are provided in correspondence to the N detection elements 22. Portions of an ultrasonic/photoacoustic wave imaging system 200 similar to those of the ultrasonic/photoacoustic wave imaging system 100 according to the aforementioned first embodiment are denoted by the same reference numerals as those in the first embodiment, and redundant description is omitted.

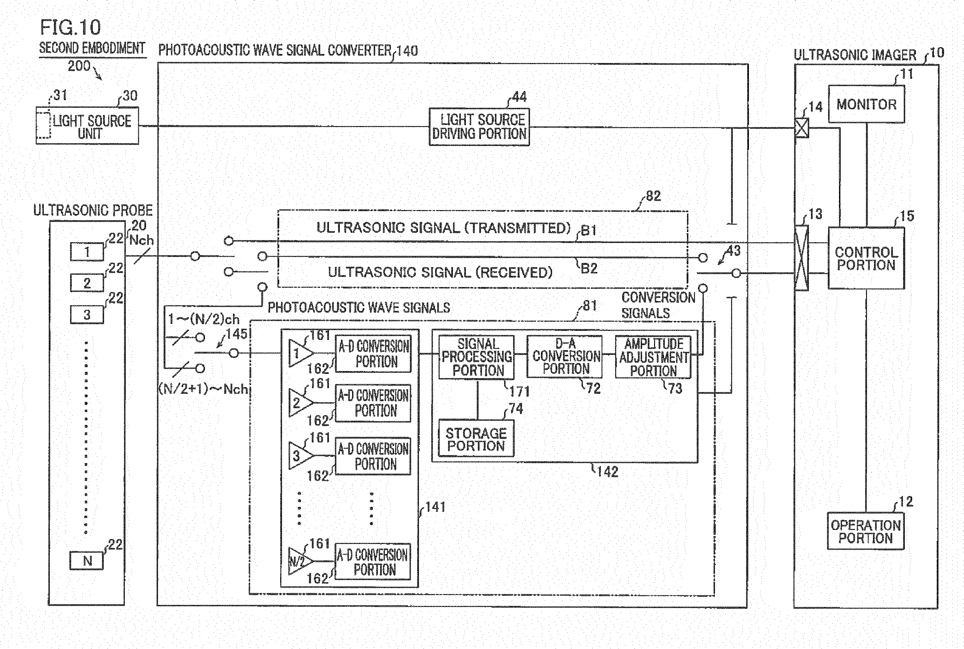

The ultrasonic/photoacoustic wave imaging system 200 (see Fig. 1) according to the second embodiment of the present invention includes an ultrasonic imager 10, an ultrasonic probe 20, a light source unit 30, and a photoacoustic wave signal converter (hereinafter referred to as the converter) 140, as shown in Fig. 10. The converter 140 includes a receiving portion 141 and a signal conversion portion 142.

According to the second embodiment, the receiving portion 141 is provided with N/2 amplification portions 161 and N/2 analog-digital conversion portions (hereinafter referred to as the A-D conversion portions) 162 with respect to N detection elements 22 of the ultrasonic probe 20. In other words, according to the second embodiment, in the receiving portion 141, the number of amplification portions 161 and the number of A-D conversion portions 162 are equal to one half the number N of detection elements 22. The remaining structures of the amplification portions 161 and the A-D conversion portions 162 are similar to those of the amplification portions 61 and the A-D conversion portions 62 according to the aforementioned first embodiment.

According to the second embodiment, the converter 140 is provided with a reception switching portion 145 configured to allow the receiving portion 141 to receive N photoacoustic wave signals detected by the N respective detection elements 22 in plural batches (in twice).

Specifically, the reception switching portion 145 is configured to allow the receiving portion 141 to receive N/2 photoacoustic wave signals detected by first to N/2th detection elements 22 of the N photoacoustic wave signals detected by the N respective detection elements 22 by certain pulse emission. Furthermore, the reception switching portion 145 is configured to allow the receiving portion 141 to receive N/2 photoacoustic wave signals detected by (N/2 + 1)^{st} to N^{th} detection elements 22 of the N photoacoustic wave signals detected by the N respective detection elements 22 by pulse emission subsequent to the certain pulse emission. The reception switching portion 145 is configured to allow the receiving portion 141 to receive the N photoacoustic wave signals detected by the N respective detection elements 22 in plural batches (in twice) by switching a signal path in this manner.

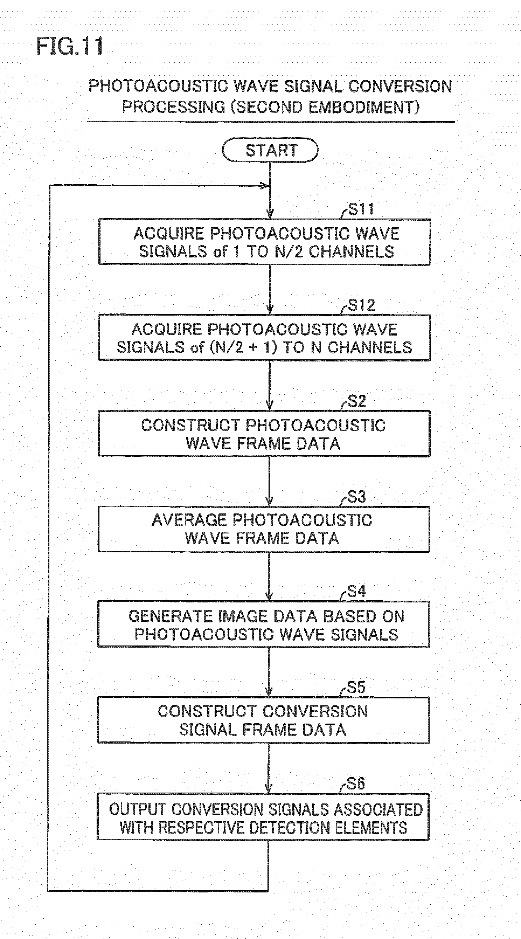

According to the second embodiment, the signal conversion portion 142 includes a signal processing portion 171 that generates conversion signals on the basis of the photoacoustic wave signals received in plural batches by the receiving portion 141. The remaining structures of the signal processing portion 171 are similar to those of the signal processing portion 71 according to the aforementioned first embodiment. Photoacoustic wave signal conversion processing performed by the signal processing portion 171 of the converter 140 according to the second embodiment is now described on the basis of a flowchart with reference to Fig. 11.

First, the signal processing portion 171 acquires N/2 photoacoustic wave signals detected by the first to N/2th detection elements 22 of N photoacoustic wave signals detected by the N respective detection elements 22 by certain pulse emission at a step S11. At this time, the signal processing portion 171 acquires the photoacoustic wave signals of the first to N/2th detection elements 22 received by the receiving portion 141 (see Fig. 10) and stored in a storage portion 74 (see Fig. 10).

Then, the signal processing portion 171 acquires N/2 photoacoustic wave signals detected by the (N/2 + 1)^{st} to N^{th} detection elements 22 of N photoacoustic wave signals detected by the N respective detection elements 22 by pulse emission subsequent to the certain pulse emission at a step S12. At this time, the signal processing portion 171 acquires the photoacoustic wave signals of the (N/2 + 1)^{st} to N^{th} detection elements 22 received by the receiving portion 141 (see Fig. 10) and stored in the storage portion 74 (see Fig. 10). In other words, the signal processing portion 171 acquires the photoacoustic wave signals of the first to N^{th} detection elements 22 by the processing at the steps S11 and S12.

Then, the signal processing portion 171 constructs photoacoustic wave frame data LD on the basis of the N photoacoustic wave signals received in twice at a step S2. Thereafter, processing at steps S3 to S6 is performed, similarly to the aforementioned first embodiment. Consequently, also according to the second embodiment, a photoacoustic wave image is generated in the ultrasonic imager 10, and the generated photoacoustic wave image is displayed on a monitor 11. Then, the signal processing portion 171 returns to the step S11 and acquires subsequent photoacoustic wave signals.

The remaining structures of the ultrasonic/photoacoustic wave imaging system 200 according to the second embodiment are similar to those of the ultrasonic/photoacoustic wave imaging system 100 according to the aforementioned first embodiment.

According to the second embodiment, the following effects can be obtained.

According to the second embodiment, as hereinabove described, the converter 140 is provided with the signal conversion portion 142 that generates the conversion signals on the basis of the photoacoustic wave signals received by the receiving portion 141 and outputs the generated conversion signals to the ultrasonic imager 10. Thus, also according to the second embodiment, the marketed ultrasonic imager 10 can serve as a photoacoustic imager and display an image based on the photoacoustic wave signals without changing imager body hardware, similarly to the aforementioned first embodiment.

According to the second embodiment, as hereinabove described, the receiving portion 141 is provided with the (N/2) amplification portions 161 and the (N/2) A-D conversion portions 162 fewer in number than a plurality of (N) detection elements 22. Furthermore, the converter 140 is provided with the reception switching portion 145 configured to allow the receiving portion 141 to receive a plurality of (N) photoacoustic wave signals detected by the plurality of (N) respective detection elements 22 in plural batches (in twice). Thus, the photoacoustic wave signals detected by the plurality of respective detection elements 22 can be reliably acquired while the structure of the receiving portion 141 is simplified.

The remaining effects of the second embodiment are similar to those of the aforementioned first embodiment.

### (Third Embodiment)

A third embodiment is now described with reference to Figs. 1, 12, and 13. In this third embodiment, two different wavelength light sources 231a and 231b are provided in a light source unit 230, unlike the aforementioned first embodiment in which one light source 31 is provided in the light source unit 30. Portions of an ultrasonic/photoacoustic wave imaging system 300 similar to those of the ultrasonic/photoacoustic wave imaging system 100 according to the aforementioned first embodiment are denoted by the same reference numerals as those in the first embodiment, and redundant description is omitted.

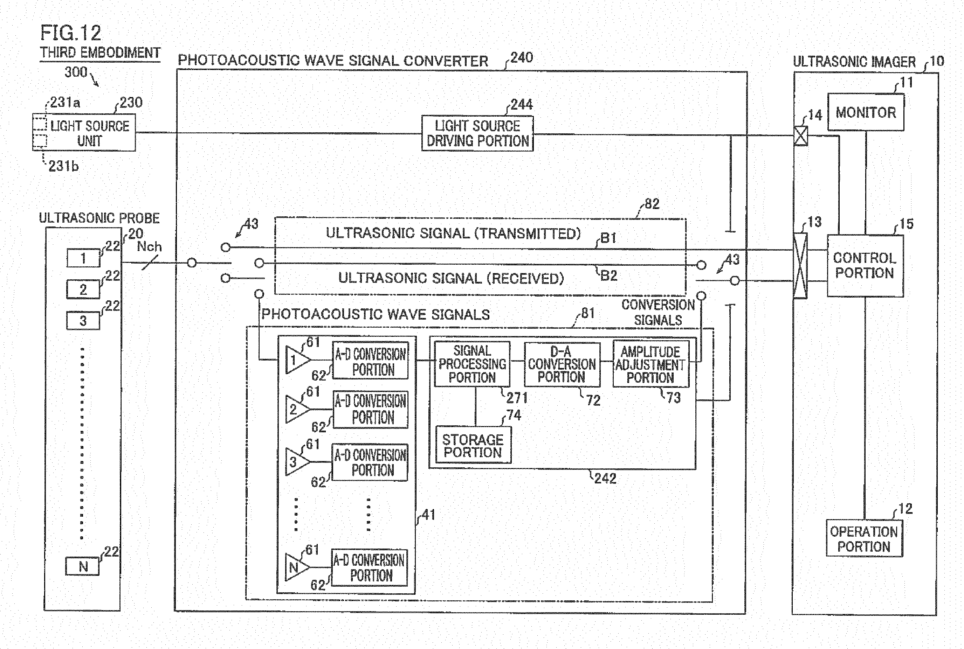

The ultrasonic/photoacoustic wave imaging system 300 (see Fig. 1) includes an ultrasonic imager 10, an ultrasonic probe 20, a light source unit 230, and a photoacoustic wave signal converter (hereinafter referred to as the converter) 240, as shown in Fig. 12. The converter 240 includes a signal conversion portion 242 and a light source driving portion 244.

According to the third embodiment, the light source unit 230 includes the light source 231a that generates light having a first wavelength and the light source 231b that generates light having a second wavelength different from the first wavelength. Both the light sources 231a and 231b are configured to generate light (light with a center wavelength at about 700 nm to about 1000 nm, for example) with a measurement wavelength of an infrared region suitable for measuring a specimen such as a human body. As the light sources 231a and 231b, semiconductor light-emitting elements such as light-emitting diode elements, semiconductor laser elements, or organic light-emitting diode elements can be employed, for example. The measurement wavelengths of the light sources 231a and 231b may be properly determined according to a detection object desired to be detected.

According to the third embodiment, the light source driving portion 244 is configured to control the light sources 231a and 231b of the light source unit 230 provided outside to emit pulsed light. The light source driving portion 244 can control the light sources 231a and 231b to alternately emit pulsed light having the first wavelength and pulsed light having the second wavelength, for example. Consequently, according to the third embodiment, the ultrasonic probe 20 detects two types of photoacoustic wave signals due to the light having the first wavelength and photoacoustic wave signals due to the light having the second wavelength.

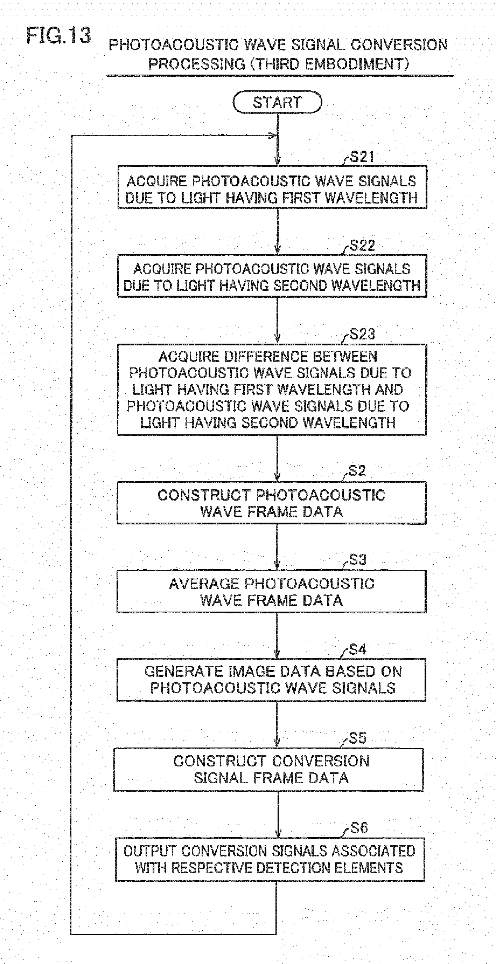

According to the third embodiment, the signal conversion portion 242 includes a signal processing portion 271 that generates conversion signals on the basis of two types of the photoacoustic wave signals due to the light having the first wavelength and the photoacoustic wave signals due to the light having the second wavelength. The remaining structures of the signal processing portion 271 are similar to those of the signal processing portion 71 according to the aforementioned first embodiment. Photoacoustic wave signal conversion processing performed by the signal processing portion 271 of the converter 240 according to the third embodiment is now described on the basis of a flowchart with reference to Fig. 13.

First, the signal processing portion 271 acquires photoacoustic wave signals due to the light having the first wavelength at a step S21. At this time, the signal processing portion 271 acquires the photoacoustic wave signals due to the light having the first wavelength received by a receiving portion 41 (see Fig. 12) and stored in a storage portion 74 (see Fig. 12).

Then, the signal processing portion 271 acquires photoacoustic wave signals due to the light having the second wavelength at a step S22. At this time, the signal processing portion 271 acquires the photoacoustic wave signals due to the light having the second wavelength received by the receiving portion 41 (see Fig. 12) and stored in the storage portion 74 (see Fig. 12).

Then, the signal processing portion 271 acquires a difference between the photoacoustic wave signals due to the light having the first wavelength and the photoacoustic wave signals due to the light having the second wavelength at a step S23. In other words, at the step S23, the signal processing portion 271 acquires the difference between the photoacoustic wave signals due to the light having the first wavelength and the photoacoustic wave signals due to the light having the second wavelength to acquire photoacoustic wave signals obtained by synthesizing the photoacoustic wave signals due to the light having the first wavelength and the photoacoustic wave signals due to the light having the second wavelength.

Then, the signal processing portion 271 constructs photoacoustic wave frame data LD on the basis of the photoacoustic wave signals acquired at the step S23. Thereafter, processing at steps S3 to S6 is performed, similarly to the aforementioned first embodiment. Consequently, also according to the third embodiment, a photoacoustic wave image is generated in the ultrasonic imager 10, and the generated photoacoustic wave image is displayed on a monitor 11. Then, the signal processing portion 271 returns to the step S21 and acquires subsequent photoacoustic wave signals (photoacoustic wave signals due to light having the first wavelength).

The remaining structures of the ultrasonic/photoacoustic wave imaging system 300 according to the third embodiment are similar to those of the ultrasonic/photoacoustic wave imaging system 100 according to the aforementioned first embodiment.

According to the third embodiment, the following effects can be obtained.

According to the third embodiment, as hereinabove described, the converter 240 is provided with the signal conversion portion 242 that generates the conversion signals on the basis of the photoacoustic wave signals received by the receiving portion 41 and outputs the generated conversion signals to the ultrasonic imager 10. Thus, also according to the third embodiment, the marketed ultrasonic imager 10 can serve as a photoacoustic imager and display a photoacoustic wave image without changing imager body hardware, similarly to the aforementioned first embodiment.

According to the third embodiment, as hereinabove described, the signal conversion portion 242 is provided with the signal processing portion 271 that generates the conversion signals on the basis of the photoacoustic wave signals due to the light having the first wavelength and the photoacoustic wave signals due to the light having the second wavelength. Thus, the conversion signals can be reliably generated also in the structure of obtaining a plurality of photoacoustic wave signals due to light having a plurality of wavelengths. Consequently, the conversion signals containing a greater variety of information in the specimen can be reliably generated, as compared with the case where the conversion signals are generated on the basis of photoacoustic wave signals due to light having a single wavelength.

The remaining effects of the third embodiment are similar to those of the aforementioned first embodiment.

### (Fourth Embodiment)

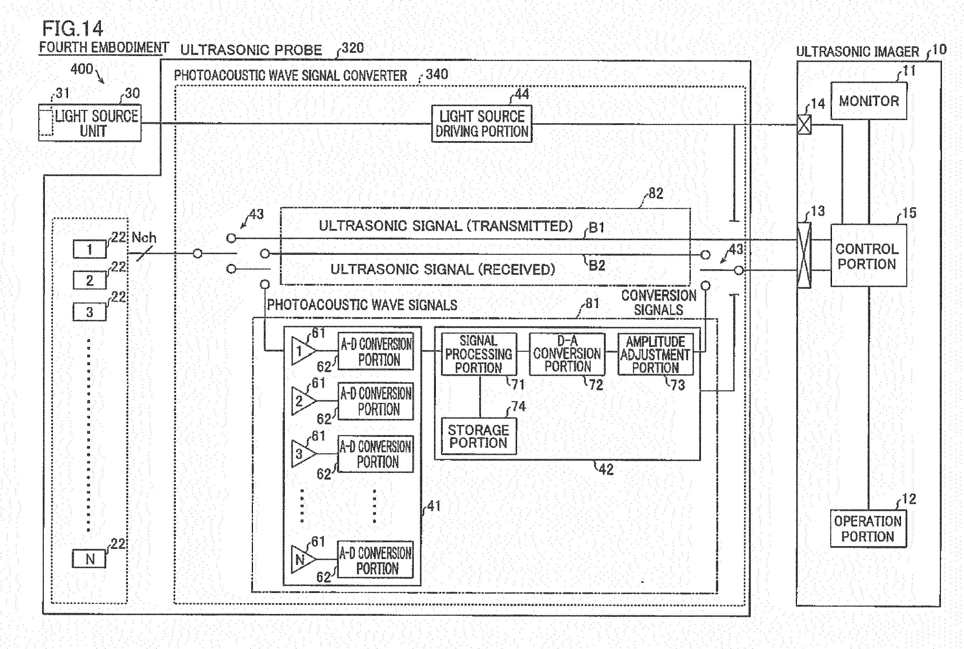

A fourth embodiment is now described with reference to Fig. 14. In this fourth embodiment, a photoacoustic wave signal converter is incorporated as a photoacoustic wave signal conversion portion 340 in an ultrasonic probe 320, unlike the aforementioned first to third embodiments. Portions of an ultrasonic/photoacoustic wave imaging system 400 similar to those of the ultrasonic/photoacoustic wave imaging system 100 according to the aforementioned first embodiment are denoted by the same reference numerals as those in the first embodiment, and redundant description is omitted.

The ultrasonic/photoacoustic wave imaging system 400 according to the fourth embodiment of the present invention includes an ultrasonic imager 10, an ultrasonic probe 320, and a light source unit 30, as shown in Fig. 14. The ultrasonic imager 10 is directly connected to the ultrasonic probe 320. According to this fourth embodiment, the photoacoustic wave signal conversion portion 340 including a receiving portion 41 and a signal conversion portion 42 is arranged in a housing of the ultrasonic probe 320. In other words, the ultrasonic probe 320 is the probe incorporating a photoacoustic wave signal converter.

In other words, according to the fourth embodiment, detection elements 22 detect photoacoustic wave signals, the receiving portion 41 receives the detected photoacoustic wave signals, and the signal conversion portion 42 converts the received photoacoustic wave signals to conversion signals in the housing of the ultrasonic probe 320. Then, the ultrasonic probe 320 outputs the conversion signals to the ultrasonic imager 10.

The remaining structures of the ultrasonic/photoacoustic wave imaging system 400 according to the fourth embodiment are similar to those of the ultrasonic/photoacoustic wave imaging system 100 according to the aforementioned first embodiment.

According to the fourth embodiment, the following effects can be obtained.

According to the fourth embodiment, as hereinabove described, the photoacoustic wave signal conversion portion 340 that generates the conversion signals on the basis of the photoacoustic wave signals received by the receiving portion 41 and includes the signal conversion portion 42 that outputs the generated conversion signals to the ultrasonic imager 10 is provided in the ultrasonic probe 320. Thus, also according to the fourth embodiment, the marketed ultrasonic imager 10 can serve as a photoacoustic imager and display a photoacoustic wave image without changing imager body hardware, similarly to the aforementioned first embodiment. Furthermore, the photoacoustic wave signal conversion portion 340 is incorporated as a photoacoustic wave signal converter in the ultrasonic probe 320, and hence the structure can be further simplified by configuring the photoacoustic wave signal converter as an apparatus integrated with the ultrasonic probe 320.

According to the fourth embodiment, the signal conversion portion 42 is configured to generate the conversion signals on the basis of the photoacoustic wave signals received by the receiving portion 41 and to output the generated conversion signals to the ultrasonic imager 10 according to the reception cycle (i.e. a transmission cycle Tb) of ultrasonic signals received by the ultrasonic imager 10, similarly to the aforementioned first embodiment. Thus, the ultrasonic imager 10 can receive the conversion signals into which the photoacoustic wave signals are converted at the timing of normally receiving the ultrasonic signals in the ultrasonic imager 10. Consequently, the ultrasonic imager 10 can more easily process the conversion signals into which the photoacoustic wave signals are converted.

According to the fourth embodiment, the signal conversion portion 42 is configured to generate the conversion signals corresponding to detection times (i.e. times T2) in which the ultrasonic signals are detected when the detection elements 22 of the ultrasonic probe 320 receive ultrasonic waves by substantially doubling detection times T1 in which the photoacoustic wave signals are detected when the detection elements 22 of the ultrasonic probe 320 receive photoacoustic wave and to output the generated conversion signals to the ultrasonic imager 10, similarly to the aforementioned first embodiment. Thus, the conversion signals obtained by associating the detection times T1 in which the photoacoustic wave signals are detected with the detection times (T2) in which the ultrasonic signals are detected can be easily generated. Furthermore, the conversion signals obtained by associating the detection times T1 in which the photoacoustic wave signals are detected with the detection times (T2) in which the ultrasonic signals are detected are input into the ultrasonic imager 10, and hence the ultrasonic imager 10 can easily process the conversion signals into which the photoacoustic wave signals are converted.

According to the fourth embodiment, the signal conversion portion 42 is provided with a signal processing portion 71 that generates the conversion signals associated with the respective detection elements 22 of the ultrasonic probe 320 on the basis of the photoacoustic wave signals received by the receiving portion 41, similarly to the aforementioned first embodiment. Thus, the conversion signals are divided with respect to the respective detection elements 22 of the ultrasonic probe 320, and hence the ultrasonic imager 10 can more easily process the conversion signals.

According to the fourth embodiment, the receiving portion 41 is provided with A-D conversion portions 62 that convert the photoacoustic wave signals from analog signals into digital signals when receiving the photoacoustic wave signals. Furthermore, the signal processing portion 71 of the signal conversion portion 42 is configured to generate the conversion signals on the basis of the photoacoustic wave signals as the digital signals converted by the A-D conversion portions 62 of the receiving portion 41. Thus, the signal processing portion 71 of the signal conversion portion 42 can process the photoacoustic wave signals as digital signals. Consequently, conditions for processing the photoacoustic wave signals by the signal processing portion 71 can be easily changed unlike the case where photoacoustic wave signals as analog signals are processed.

The remaining effects of the fourth embodiment are similar to those of the aforementioned first embodiment.

The embodiments disclosed this time must be considered as illustrative in all points and not restrictive. The range of the present invention is shown not by the above description of the embodiments but by the scope of claims for patent, and all modifications within the meaning and range equivalent to the scope of claims for patent are further included.

For example, while the converter 40 (140, 240) and the photoacoustic wave signal conversion portion 340 are configured to convert the photoacoustic wave signals as digital signals into the conversion signals in each of the aforementioned first to fourth embodiments, the present invention is not restricted to this. According to the present invention, the converter may alternatively be configured to convert photoacoustic wave signals as analog signals into conversion signals.

While the switching portions 43 are provided to selectively switch between the first signal path 81 and the second signal path 82, and the converter 40 (140, 240) and the photoacoustic wave signal conversion portion 340 are configured to be capable of switching between detection of the photoacoustic wave signals and detection of the ultrasonic signals in each of the aforementioned first to fourth embodiments, the present invention is not restricted to this. According to the present invention, the converter may alternatively be configured to convert only the photoacoustic wave signals through the first signal path 81 without providing the second signal path 82. In this case, the ultrasonic signals may be detected by direct connection between the ultrasonic imager 10 and the ultrasonic probe 20.

While the light source driving portion 44 (244) configured to drive the light source unit 30 (230) is provided in the converter 40 (140, 240) and the photoacoustic wave signal conversion portion 340 in each of the aforementioned first to fourth embodiments, the present invention is not restricted to this. According to the present invention, no light source driving portion may alternatively be provided in the converter. In this case, the light source driving portion may be provided in the light source unit or may be provided as a separate apparatus.

While the amplitude adjustment portion 73 is provided in the converter 40 (140, 240) and the photoacoustic wave signal conversion portion 340 in each of the aforementioned first to fourth embodiments, the present invention is not restricted to this. According to the present invention, no amplitude adjustment portion may alternatively be provided in the converter. In this case, the amplitude may be adjusted in the ultrasonic imager 10.

While the receiving portion 141 is provided with the N/2 amplification portions 161 and the N/2 A-D conversion portions 162 with respect to the N detection elements 22 in the aforementioned second embodiment, the present invention is not restricted to this. According to the present invention, the receiving portion may alternatively be provided with more or less than N/2 amplification portions and N/2 A-D conversion portions such as N/3 or N/4 amplification portions and A-D conversion portions with respect to the N detection elements 22. In this case, the N photoacoustic wave signals may be received by the receiving portion in corresponding batches.

While the signal processing portion 271 that generates the conversion signals on the basis of two types of the photoacoustic wave signals due to the light having the first wavelength and the photoacoustic wave signals due to the light having the second wavelength is provided in the signal conversion portion 242 in the aforementioned third embodiment, the present invention is not restricted to this. According to the present invention, the signal processing portion may alternatively be configured to generate conversion signals on the basis of three or more types of photoacoustic wave signals due to light having three or more wavelengths in the case where the light source unit is configured to be capable of applying the light having the three or more wavelengths.

While the processing operations performed by the signal processing portion 71 (171, 271) according to the present invention are described, using the flowcharts described in a flow-driven manner in which processing is performed in order along a processing flow for the convenience of illustration in each of the aforementioned first to third embodiments, the present invention is not restricted to this. According to the present invention, the processing operations performed by the signal processing portion 71 (171, 271) may alternatively be performed in an event-driven manner in which processing is performed on an event basis. In this case, the processing operations performed by the signal processing portion 71 (171, 271) may be performed in a complete event-driven manner or in a combination of an event-driven manner and a flow-driven manner.

## Claims

1. A photoacoustic wave signal converter (40, 140, 240, 340) comprising:
a receiving portion (41, 141) that receives a photoacoustic wave signal generated from a detection object (Q) in a specimen (P) that has absorbed light, detected by an ultrasonic probe (20, 320); and
a signal conversion portion (42, 142, 242) that generates a conversion signal corresponding to an ultrasonic signal generated when the ultrasonic probe receives an ultrasonic wave on the basis of the photoacoustic wave signal received by the receiving portion and outputs the conversion signal that is generated to an ultrasonic imager (10).

2. The photoacoustic wave signal converter according to claim 1, wherein
the signal conversion portion is configured to generate the conversion signal on the basis of the photoacoustic wave signal received by the receiving portion and to output the conversion signal that is generated to the ultrasonic imager according to a reception cycle of the ultrasonic signal received by the ultrasonic imager.

3. The photoacoustic wave signal converter according to claim 1 or 2, wherein
the signal conversion portion is configured to generate the conversion signal corresponding to a detection time in which the ultrasonic signal is detected when the ultrasonic probe receives the ultrasonic wave by doubling a detection time in which the photoacoustic wave signal is detected when the ultrasonic probe receives a photoacoustic wave and to output the conversion signal that is generated to the ultrasonic imager.

4. The photoacoustic wave signal converter according to any of claims 1 to 3, wherein
the signal conversion portion includes a signal processing portion (71, 171, 271) that generates the conversion signal associated with each of a plurality of detection elements (22) of the ultrasonic probe on the basis of the photoacoustic wave signal received by the receiving portion.

5. The photoacoustic wave signal converter according to claim 4, wherein
the receiving portion includes an analog-digital conversion portion (62, 162) that converts the photoacoustic wave signal from an analog signal into a digital signal when receiving the photoacoustic wave signal, and
the signal processing portion of the signal conversion portion is configured to generate the conversion signal on the basis of the photoacoustic wave signal as the digital signal converted by the analog-digital conversion portion of the receiving portion.

6. The photoacoustic wave signal converter according to claim 5, wherein
the receiving portion further includes an amplification portion (61, 161) that amplifies the photoacoustic wave signal, and
the analog-digital conversion portion of the receiving portion is configured to convert the photoacoustic wave signal having been amplified by the amplification portion from an analog signal into a digital signal.

7. The photoacoustic wave signal converter according to claim 6, wherein
the signal conversion portion further includes a signal adjustment portion (72, 73) that adjusts an amplitude of the conversion signal by converting the conversion signal output from the signal processing portion of the signal conversion portion from a digital signal into an analog signal and multiplying the conversion signal converted into the analog signal by an amplification smaller than 1.

8. The photoacoustic wave signal converter according to claim 6 or 7, wherein
the receiving portion (41) includes a plurality of analog-digital conversion portions (62) and a plurality of amplification portions (61) equal in number to the plurality of detection elements (22) of the ultrasonic probe (20).

9. The photoacoustic wave signal converter according to claim 6 or 7, wherein
the receiving portion (141) includes the analog-digital conversion portion (162) and the amplification portion (161) fewer in number than the plurality of detection elements (22) of the ultrasonic probe (20),
the photoacoustic wave signal converter further comprising a reception switching portion (145) configured to allow the receiving portion to receive photoacoustic wave signals detected by the detection elements of the ultrasonic probe in plural batches.

10. The photoacoustic wave signal converter according to any of claims 1 to 9, further comprising a switching portion (43) capable of selectively switching between a first signal path (81) for receiving the photoacoustic wave signal through the receiving portion and outputting the conversion signal to the ultrasonic imager when the ultrasonic probe detects the photoacoustic wave signal and a second signal path (82) for outputting the ultrasonic signal to the ultrasonic imager not through the receiving portion when the ultrasonic probe detects the ultrasonic signal.

11. The photoacoustic wave signal converter according to any of claims 1 to 10, further comprising a light source driving portion (44, 244) configured to control a light source (31, 231a, 231b) provided outside in order to generate the photoacoustic wave signal to emit pulsed light.

12. The photoacoustic wave signal converter according to claim 11, wherein
the light source driving portion is configured to control the light source including at least one of a light-emitting diode element, a semiconductor laser element, and an organic light-emitting diode element to emit the pulsed light.

13. The photoacoustic wave signal converter according to claim 11 or 12, wherein
the light source driving portion is configured to be capable of adjusting a current value for controlling the light source provided outside in order to generate the photoacoustic wave signal to emit the pulsed light on the basis of a control signal from the ultrasonic imager.

14. The photoacoustic wave signal converter according to any of claims 1 to 13, wherein
the photoacoustic wave signal converter (40, 140, 240) comprising the receiving portion and the signal conversion portion is arranged between the ultrasonic probe (20) and the ultrasonic imager (10).

15. The photoacoustic wave signal converter according to any of claims 1 to 14, wherein
the photoacoustic wave signal converter (340) comprising the receiving portion and the signal conversion portion is incorporated in the ultrasonic probe (320).
